# FASCICULE DE BREVET EUROPEEN

(11) **EP 3 315 965 B1**
(45) Date de publication et mention de la délivrance du brevet: **15.05.2019**
(21) Numéro de dépôt: 17198626.8
(22) Date de dépôt: 26.10.2017
(51) Int. Cl.: G01N 33/497

(54) **DISPOSITIF POUR MESURER LES QUANTITES DE GAZ REJETEES LORS DES ERUCTATIONS D'UN ANIMAL**
VORRICHTUNG ZUM MESSEN DER GASMENGEN, DIE BEIM AUFSTOSSEN EINES TIERES AUSGESTOSSEN WERDEN
DEVICE FOR MEASURING THE AMOUNTS OF GASES EJECTED WHEN AN ANIMAL BELCHES

(30) Priorité: 27.10.2016 FR 1660425
(43) Date de publication de la demande: 02.05.2018
(73) Titulaire: AGRIAL, 14000 Caen (FR)
(72) Inventeur: GIGER, Amélie, 14000 CAEN (FR)
(74) Mandataire: Cabinet Le Guen Maillet

(56) Documents cités:
- DE-C1- 19 960 257
- US-A- 5 265 618
- US-A1- 2015 285 783
- RAGHAVENDRA BHATTA ET AL: "Measurement of methane production from ruminants", ASIAN-AUSTRALASIAN JOURNAL OF ANIMAL SCIENCES, SUWEON, KR, vol. 20, no. 8, août 2007 (2007-08), pages 1305-1318, XP002691339, ISSN: 1011-2367, DOI: 10.5713/AJAS.2007.1305

## Description

La présente invention concerne un dispositif et un procédé pour mesurer les quantités de gaz, notamment les gaz à effet de serre, rejetées par un animal et plus particulièrement lors des éructations d'un animal, tel qu'un ruminant, notamment un bovin.

Le rejet des gaz à effet de serre dans l'atmosphère joue un rôle important dans le changement climatique.

L'élevage apporte une contribution non négligeable au rejet de gaz à effet de serre, comme le dioxyde de carbone et en particulier le méthane.

L'impact du méthane sur le réchauffement climatique global est de 20 à 25 fois supérieur à celui du dioxyde de carbone. La plus grande partie des émissions de méthane est causée par les éructations des ruminants.

Une vache moyenne émet environ entre 500 et 600 litres de méthane par jour. Le méthane est produit par les ruminants comme un sous-produit de la fermentation de la masse organique dans l'intestin.

La vache relâche du méthane par la bouche et les naseaux lorsqu'elle mange. Plus de 90% du méthane rejeté par la vache l'est en fait par la tête de la vache lorsqu'elle éructe.

La vache éructe lorsqu'elle avale de la nourriture tête baissée et lorsqu'elle broute. Par contre, elle n'éructe pas lorsqu'elle rumine.

Il est souhaitable de trouver un moyen simple pour mesurer les émissions de méthane éructées par la vache dans son environnement de production sans gêner l'animal.

Ces mesures pourront permettre ensuite d'adapter la nourriture du ruminant de façon à ce qu'il rejette moins de méthane.

On connait du brevet US5265618 un procédé et un système de mesure de gaz à effet de serre émis par la vache. Un tube de gaz traceur (SF₆) est avalé par l'animal. Le tube est prévu pour fuir et émettre du gaz traceur. Un système d'échantillonnage est fixé à proximité de la bouche de l'animal pour prélever des échantillons d'haleine par l'intermédiaire d'un tube d'échantillonnage. L'émission de gaz à effet de serre peut être déterminée par l'analyse des échantillons d'haleine, dans lesquels les proportions de gaz à effet de serre et de gaz traceur par rapport à l'air ambiant sont connues. Cependant, cette méthode est contraignante pour l'animal car intrusive.

On connait également du document US2015/0285783, une méthode consistant à mesurer le son émis par les éructations en sortie de bouche de la vache. Il est ainsi possible de mesurer la durée des éructations et d'estimer les quantités de méthane émises.

Cette méthode est néanmoins indirecte et peu précise.

On connait également le système commercialisé sous la marque GreenFeed (C-Lock Inc., Rapid City, USA) permettant d'estimer les émissions de méthane chez les bovins dans différentes situations d'élevage, c'est-à-dire en bâtiment et en pâturage, tout en limitant l'intervention humaine qui pourrait modifier le comportement de l'animal.

Le procédé mis en oeuvre par ce système consiste à utiliser un appât alimentaire pour attirer l'animal dans un compartiment de mesure et à distribuer de l'alimentation calibrée pour conserver l'animal au moins 5 minutes.

Les gaz émis par éructation sont échantillonnés grâce à un système d'aspiration verticale. La répétabilité de la mesure est de 70% après au moins 50 passages de l'animal (3 à 4 visites par jour sur 2 semaines). La précision de la concentration en CH₄ est de 4,5% environ.

Cependant, cette méthode est difficile à mettre en oeuvre et ne permet pas de mesurer les taux ou concentration de méthane dans les conditions de vie réelles de la vache, c'est-à-dire en parcours libre. La mesure n'est pas non plus réalisée en continu sur le lieu de production de l'animal. De plus, le coût du système GreenFeed est très élevé.

Le document RAGHAVENDRA BHATTA et al., "Measurement of methane production from ruminants", ASIAN-AUSTRALASIAN JOURNAL OF ANIMAL SCIENCES, SUWEON, KR, vol. 20, no. 8, août 2007, pages 1305-1318, XP002691339, ISSN: 1011-2367, DOI: 10.5713/ AJAS.2007.1305 et le brevet DE19960257C1 divulguent un dispositif permettant de quantifier les gaz rejetés lors des éructations d'un animal, comprenant un masque avec une jupe enfermant complètement le museau.

L'invention a donc pour objectif de pallier ces inconvénients de l'art antérieur en proposant un dispositif et un procédé pour mesurer les quantités de gaz, tels que les gaz à effet de serre, rejetées lors des éructations d'un animal, en particulier d'un ruminant, notamment en parcours libre, qui soit simple d'utilisation, non invasif, précis et efficace, et qui ne perturbe pas l'animal notamment lorsqu'il se déplace ou qu'il mange.
- A cet effet, l'invention concerne un dispositif pour mesurer les quantités de gaz rejetées lors des éructations d'un animal, le dispositif comprenant un appareil de mesure des quantités de gaz rejetées par l'animal, des moyens de fixation dudit dispositif sur le museau de l'animal, et une jupe apte à être positionnée au-dessus du museau de l'animal, la jupe comprenant des moyens pour collecter le gaz ressortant par la bouche de l'animal et pour l'acheminer jusqu'à l'appareil de mesure, le dispositif étant caractérisé en ce que la jupe présente une forme générale de U inversé et entoure seulement le dessus et les flancs du museau de l'animal tout en étant ouverte devant les nasaux et la bouche et sous le museau.

Selon un mode de réalisation de l'invention, la jupe comprend une partie supérieure arquée prolongée par deux parties latérales opposées, la partie supérieure arquée s'étendant depuis le voisinage des yeux de l'animal jusqu'au voisinage de l'extrémité du museau, et les deux parties latérales se prolongeant depuis la partie supérieure arquée jusqu'au voisinage de la partie inférieure du museau de l'animal (2) lorsque le dispositif est positionné sur l'animal.

Avantageusement, la partie supérieure arquée de la jupe comprend un logement destiné à recevoir l'appareil de mesure et la jupe comprend un orifice débouchant dans le logement (12) et permettant de canaliser le gaz d'une zone inférieure (14) située en dessous de la jupe (6) vers le logement.

Selon un mode de réalisation de l'invention, le logement est délimité par une enveloppe de protection comprenant une ouverture pour l'introduction de l'appareil de mesure.

Préférentiellement, l'enveloppe de protection comprend une sangle fixée au voisinage de l'ouverture de l'enveloppe de protection pour maintenir l'appareil de mesure dans le logement.

Selon un mode de réalisation de l'invention, le dispositif comprend une lèvre d'étanchéité positionnée sur une surface interne de la jupe, la lèvre d'étanchéité longeant un rebord interne de la jupe positionné au voisinage des yeux de l'animal, cette lèvre étant destinée à venir en appui sur le museau de l'animal pour empêcher le passage du gaz de la zone inférieure située en dessous de la jupe vers une zone supérieure positionnée au-dessus de la jupe.

Selon un mode de réalisation de l'invention, les moyens de fixation comprennent un élément de fixation, tel qu'une ou plusieurs sangles, pour fixer le dispositif sur la tête de l'animal, ainsi qu'un guide pour cet élément de fixation longeant le rebord interne de la jupe d'une extrémité d'une partie latérale de la jupe à l'autre.

Avantageusement, l'élément de fixation comprend une première sangle qui est logée dans le guide et dont les extrémités sont destinées à se rejoindre de manière à ce que ladite sangle entoure le museau de l'animal et une seconde sangle dont deux portions s'étendent perpendiculairement à la première sangle et sont prévues pour être reliées l'une à l'autre à l'arrière de la tête de l'animal.

Selon un mode de réalisation, l'appareil de mesure comprend un capteur résistif ou à infrarouge mesurant les quantités de gaz émis par l'animal.

Avantageusement, le dispositif comprend un boîtier électronique relié électriquement à l'appareil de mesure pour collecter les données de mesures enregistrées par l'appareil de mesure. Le boîtier électronique comprend un moyen de communication sans fil pour transmettre lesdites données de mesures à une base de données distante. Le boîtier électronique est destiné à être positionné sous le museau de l'animal.

Avantageusement encore, le dispositif comprend un gyroscope et/ou un inclinomètre pour déterminer l'inclinaison de la tête de l'animal afin de déclencher ou arrêter l'appareil de mesure.

L'invention concerne également un procédé pour mesurer les quantités de gaz rejetées lors des éructations d'un animal au moyen d'un dispositif tel que défini précédemment.

Selon l'invention, le procédé comprend les étapes suivantes :
- collecte du gaz rejeté par l'animal dans une zone inférieure située sous la jupe,
- canalisation du gaz collecté de la zone inférieure vers l'appareil de mesure, et
- mesure des quantités de gaz par l'appareil de mesure.

Selon une variante, le procédé comprend une étape de détection de l'inclinaison de la tête de l'animal pour déclencher ou arrêter la mesure.

Selon une autre variante, la détection de l'inclinaison de la tête étant réalisée par un gyroscope et/ou inclinomètre positionné sur le dispositif, la mesure des quantités de gaz rejetées est déclenchée lorsque la tête est en position sensiblement verticale et arrêtée lorsque la tête est inclinée par rapport à la verticale.

L'invention permet ainsi de fournir un dispositif et un procédé pour mesurer les quantités de gaz à effet de serre rejetées lors des éructations d'un ruminant en parcours libre permettant de ne pas gêner l'animal dans son lieu de vie.

Le dispositif présente une forme ergonomique adaptée à la forme du museau de la vache.

Il ne gêne pas l'animal dans le lieu de production (pâturage) notamment lorsqu'il broute de l'herbe.

Sa forme est aussi adaptée pour ne pas gêner la vision de l'animal.

Le dispositif est adapté aux contraintes d'usages. En effet, il existe des zones de chocs et de contacts autour de la tête du ruminant.

Ces zones de contact sont générées lors de l'usage de brosses à rouleau pour le massage du ruminant, des frottements dans le lieu de vie (herbe, boue, eau) et des chocs avec d'autres ruminants, par exemple.

Le dispositif et le procédé de l'invention permettent ainsi de mesurer simplement les quantités ou taux de méthane (CH₄) émis par un ruminant en condition réelle.

Cette méthode est non intrusive et efficace car la mesure est seulement réalisée lors de l'émission du méthane lorsque le ruminant mange. Il est ainsi possible d'économiser la batterie de l'appareil de mesure et d'augmenter la durée de l'expérience.

Elle permet de mieux connaître le taux de méthane rejeté lorsque le ruminant est dans son milieu de vie, ce qui est capital pour pouvoir adapter sa nourriture afin de réduire l'émission du méthane.

Les mesures effectuées pourront ensuite être corrélées avec le système « Green Feed™», par exemple.

Le dispositif peut être utilisé pour mesurer d'autres gaz comme le CO₂, par exemple. Il peut être utilisé pour des vaches, boeuf, taureau, chèvres, mouton, par exemple

Les caractéristiques de l'invention seront décrites plus en détail en se référant aux dessins annexés dans lesquels :
- la Fig. 1 représente schématiquement un dispositif pour mesurer les quantités de gaz rejetées lors des éructations d'un animal, disposé sur le museau d'un ruminant et vu de profil, selon un mode de réalisation de l'invention;
- la Fig. 2 représente schématiquement une vue montrant le dessous du dispositif selon l'invention ;
- les Figs. 3 et 4 représentent schématiquement une vue de face d'un ruminant sur lequel est fixé un dispositif selon l'invention;
- les Figs. 5 et 6 représentent schématiquement des vues de profil d'un ruminant sur lequel est fixé un dispositif selon l'invention, lorsque le ruminant abaisse la tête (Fig. 5) et la relève (Fig. 6) ;

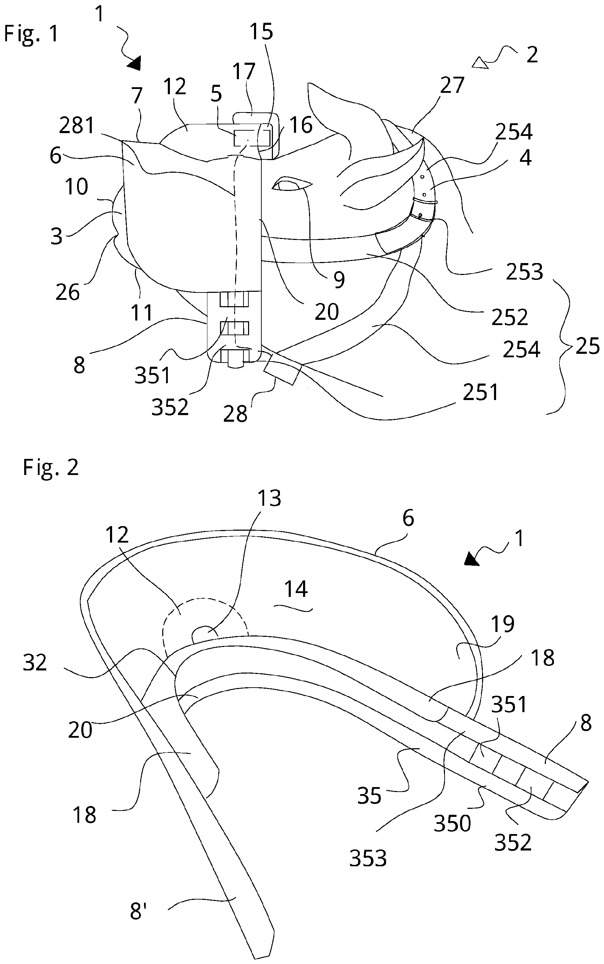

La figure 1 représente schématiquement un dispositif 1 disposé sur le museau 3 d'un ruminant 2 selon un mode de réalisation possible.

Le dispositif 1 est fixé sur le museau 3 du ruminant 2 par des moyens de fixation 4.

Le dispositif 1 comprend un appareil de mesure 5 pour mesurer les quantités de gaz, tel que le méthane, rejetées par le ruminant 2.

Selon l'invention, le dispositif 1 comprend une jupe 6 destinée à être positionnée au-dessus du museau 3 du ruminant 2 pour collecter le gaz ressortant par la bouche 26 du ruminant 2 lors de ses éructations.

L'appareil de mesure 5 est disposé sur la jupe 6.

La jupe 6 présente une forme générale de U inversé lorsqu'elle est positionnée sur le museau 3 du ruminant 2. Dit autrement, la jupe 6 présente une forme arquée ou de voûte.

La jupe 6 est constituée d'un matériau souple et déformable pour épouser plus facilement la forme du museau 3.

Le matériau souple peut être en polymère tel du caoutchouc, du polyuréthane souple, de la résine, par exemple.

La jupe 6 comprend une partie supérieure arquée 7 prolongée par deux parties latérales 8, 8' opposées (Figs. 1 et 2).

La partie supérieure arquée 7 s'étend depuis le voisinage des yeux 9 du ruminant 2 jusqu'au voisinage de l'extrémité 10 du museau 3 du ruminant 2 lorsque le dispositif 1 est positionné sur le ruminant 2.

La partie supérieure arquée 7 peut s'arrêter avant l'extrémité 10 du museau 3 ou s'étendre après celle-ci.

Les deux parties latérales 8, 8' se prolongent depuis la partie supérieure arquée 7 en direction de la partie inférieure 11 du museau 3 du ruminant 2 lorsque le dispositif 1 est positionné sur le ruminant 2. La partie inférieure 11 est en regard de la gorge.

Les deux parties latérales 8, 8' peuvent s'étendre jusqu'à la partie inférieure 11 du museau 3 ou au-dessous de cette dernière.

La jupe 6 entoure seulement le dessus et les flancs du museau 3 en étant ouverte devant les nasaux et la bouche 26 du ruminant 2 et sous le museau 3. La partie inférieure 11 du museau 3 du ruminant 2 n'est pas entourée par la jupe 6, ce qui permet à l'animal de brouter de l'herbe sans être gêné.

La partie supérieure arquée 7 de la jupe 6 comprend un logement 12 destiné à recevoir l'appareil de mesure 5. Le logement 12 fait saillie sur la surface de la partie supérieure arquée 7 de la jupe 6.

La jupe 6 comprend un orifice 13, comme représenté sur la figure 2, débouchant dans le logement 12 et permettant le passage du gaz d'une zone inférieure 14 située en dessous de la jupe 6 vers le logement 12 et l'appareil de mesure 5.

En variante, la jupe 6 peut comprendre plusieurs orifices 13.

Le logement 12 comprend une enveloppe de protection 15 comprenant une ouverture pour l'introduction de l'appareil de mesure 5.

Le logement 12 est préférentiellement intégré à la jupe 6 et peut être obtenu par moulage en même temps que la jupe 6. L'enveloppe de protection 15 est avantageusement constituée du même matériau que celui de la jupe 6.

Lorsque le dispositif 1 est positionné sur le museau 3 du ruminant 2, le logement 12 est placé sur le museau 3, entre les yeux 9 du ruminant 2. Le logement 12 est de préférence centré par rapport aux deux yeux 9 (Fig. 1).

Une ouverture 16 pour la mise en place de l'appareil de mesure 5 dans le logement est prévue et est tournée vers les yeux 9.

Le logement 12 présente par exemple une forme allongée et s'étend selon une direction parallèle au museau 3.

L'ouverture 16 est en limite du rebord interne 20 de la jupe 6, dans cet exemple. L'ouverture 16 pourrait être placée en retrait.

Le logement 12 et l'orifice 13 sont centrés par rapport aux deux parties latérales 8, 8' de la jupe 6.

Le méthane est plus léger que l'air et monte à 8m/sec. Il se comporte plus ou moins comme une "bulle" dans l'air. Lorsque le ruminant 2 broute de l'herbe, il a la tête baissé. Le méthane remonte donc à partir de la bouche 26 ou des naseaux du ruminant 2 pour être piégé par la jupe 6, dans la zone inférieure 14.

Le méthane collecté remonte ensuite dans le logement 12 à travers l'orifice 13 pour être mesuré par l'appareil de mesure 5 disposé dans le logement 12. Le méthane collecté est en fait canalisé par l'orifice 13.

L'appareil de mesure 5 comprend, par exemple, un capteur résistif ou à infrarouge (non visible sur les figures) mesurant les quantités de méthane émises par le ruminant.

Avantageusement, le capteur de méthane ou l'élément sensible de l'appareil de mesure 5 est positionné face à l'orifice 13.

Cette position du logement 12 juste au-dessus de la bouche 26 du ruminant 2 est donc avantageuse puisqu'elle permet à l'appareil de mesure 5 de capter directement et rapidement le méthane.

L'appareil de mesure 5 est maintenu dans le logement 12 au moyen d'une sangle 17, par exemple du type ayant des premières extrémités respectives fixées de part et d'autre de l'ouverture 16 et des secondes extrémités respectives reliées l'une à l'autre au moyen d'une boucle. Tout ou partie de la sangle 17 peut être élastique.

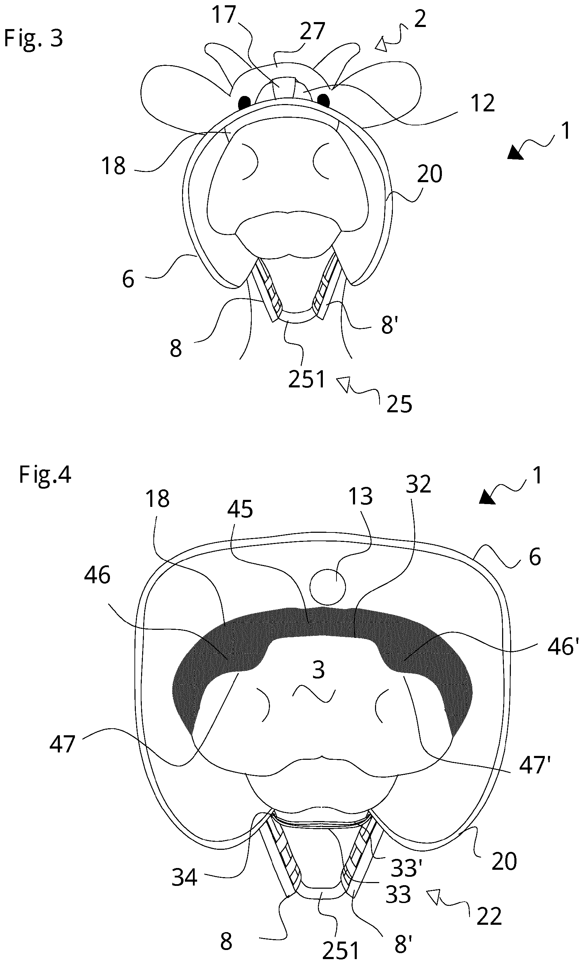

Le dispositif 1 comprend une lèvre d'étanchéité 18 positionnée sur une surface interne 19 de la jupe 6, comme représenté notamment sur les figures 2 et 4.

La lèvre d'étanchéité 18 longe la proximité du rebord interne 20 de la jupe 6 au moins sur la partie de ce rebord 20 qui est positionné au voisinage des yeux 9 du ruminant 2 lorsque le dispositif 1 est fixé sur l'animal. La lèvre d'étanchéité 18 est positionnée à quelques centimètres du rebord interne 20 entre celui-ci et l'orifice 13.

La lèvre d'étanchéité 18 est destinée à venir en appui sur le museau 3 du ruminant 2 pour empêcher la fuite du gaz de la zone inférieure 14 située en dessous de la jupe 6 vers l'extérieur de la jupe 6 et au-delà du rebord interne 20.

La lèvre d'étanchéité 18 comprend une surface d'appui 32 qui vient en contact avec le museau 3 du ruminant 2 et épouse la surface du museau 3.

La lèvre d'étanchéité 18 est constituée d'un matériau souple qui peut être le même que celui de la jupe 6. Elle peut être obtenue lors du moulage du dispositif 1.

La lèvre d'étanchéité 18 peut présenter une forme régulière.

Selon un autre mode de réalisation possible, la lèvre d'étanchéité 18 peut présenter une forme ondulée (en forme de vague) pour épouser la forme irrégulière du museau 3, comme représenté sur la figure 4.

La lèvre d'étanchéité 18 comprend alors une zone centrale 45 de faible largeur en contact avec le dessus du museau 3 du ruminant 2. La lèvre d'étanchéité 18 en forme de vague comprend également deux zones latérales 46, 46' disposées de part et d'autre de la zone centrale 45 épousant les creux 47, 47' formés sur le museau 3.

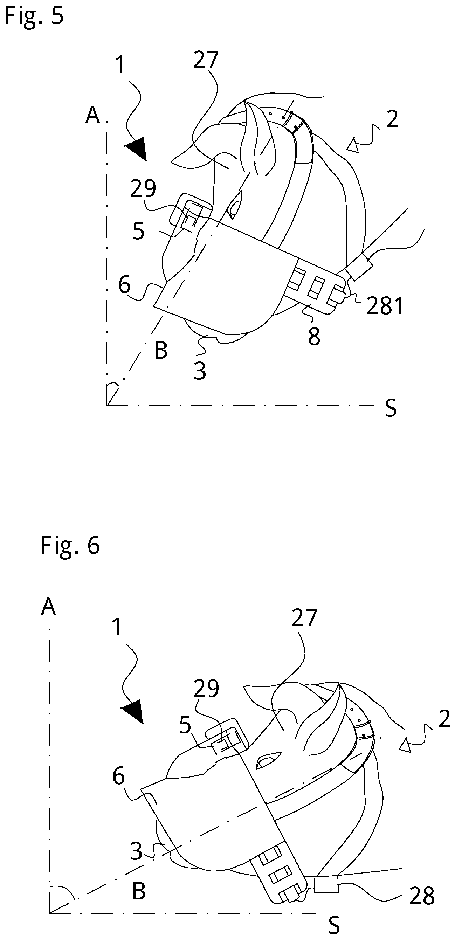

Le dispositif 1 comprend un moyen de serrage 22 pour serrer la lèvre d'étanchéité 18 contre le museau 3 du ruminant 2, comme représenté sur la figure 5.

Le moyen de serrage 22 est destiné à être positionné sous le museau 3 du ruminant 2 et à plaquer la lèvre d'étanchéité 18 contre le museau 3.

Par exemple, le moyen de serrage 22 peut comprendre deux élastiques 33, 33' s'insérant dans des orifices, par exemple 34, aménagés dans les parties latérales 8, 8' de la jupe 6.

Une fois serrés, les deux élastiques 33 peuvent en outre être bloqués par des noeuds, ou équivalent, venant en butée contre la surface externe des parties latérales 8 entourant les orifices 34.

Les moyens de fixation 4 comprennent un guide 35, longeant le rebord interne 20 de la jupe 6 d'une extrémité d'une partie latérale (8) de la jupe (6) à l'autre (8'), comme représenté sur les figures 1 et 2, et permettant le passage, le maintien et le guidage d'un élément de fixation 25 du dispositif 1 sur l'animal.

Le guide 35 présente une tenue mécanique supérieure à celle de la jupe 6. Il est composé de parois telles que les parois 350, 351, 352 et 353.

Les moyens de fixation 4 comprennent ainsi un élément de fixation 25, lequel comprend une première sangle 251 qui est logée dans le guide 35 et passe sous les parois 351, 352 du guide 35. Ses extrémités sont destinées à se rejoindre de manière à ce que la sangle 251 entoure le museau 3 de l'animal en passant sous la gorge. Une seconde sangle est également prévue, dont deux portions 252, 254 s'étendent perpendiculairement à la première sangle 251 et sont reliées l'une à l'autre à l'arrière de la tête de l'animal, par exemple derrière les oreilles ou les cornes. Les portions de sangle 252, 254 et les extrémités de la sangle 251 sont reliées par des boucles telle que la boucle 253. Bien entendu, la longueur des sangles est réglable. Avantageusement les sangles peuvent être de type licol.

Le dispositif 1 comprend encore un boitier électronique 28 relié électriquement par un fil 281 à l'appareil de mesure 5 pour collecter les données de mesures enregistrées par l'appareil de mesure 5, comme représenté sur les figures 1, 5 et 6.

Le boîtier électronique 28 comprend un moyen de communication sans fil pour transmettre les données de mesures à une base de données distante.

La communication sans fil peut se faire par Bluetooth, infrarouge ou par ondes électromagnétiques.

Le boîtier électronique 28 est positionné sous le museau 3 du ruminant 2. Il est ainsi protégé des chocs lorsque les ruminants s'entrechoquent. Il est attaché par l'intermédiaire d'une sangle 254, laquelle peut être reliée à la sangle 252.

L'appareil de mesure 5 transmet les données de mesures à une base distante qui peut être un ordinateur, une tablette ou un téléphone portable via une application spécifique.

Le dispositif 1 comprend un gyroscope et/ou un inclinomètre 29 pour déterminer l'inclinaison de la tête du ruminant 2 afin de déclencher ou arrêter l'appareil de mesure 5.

Le gyroscope et/ou l'inclinomètre 29 est de préférence intégré dans l'appareil de mesure 5.

L'invention concerne également un procédé pour mesurer les quantités de gaz à effet de serre rejetées lors des éructations du ruminant 2 au moyen du dispositif 1 tel que défini précédemment.

Le procédé comprend une étape de collecte du gaz rejeté par le ruminant 2 dans une zone inférieure 14 située sous la jupe 6, une étape de transfert ou canalisation du gaz à effet de serre collecté vers l'appareil de mesure 5 positionné dans une zone supérieure située au-dessus de la jupe 6 et une étape de mesure des quantités de gaz par l'appareil de mesure 5.

Le procédé comprend également une étape de détection de l'inclinaison de la tête 27 du ruminant 2 pour déclencher ou arrêter la mesure.

La détection de l'inclinaison de la tête 27 est réalisée par le gyroscope et/ou l'inclinomètre 29.

La mesure des quantités de gaz est déclenchée lorsque la tête 27 du ruminant 2 est en position sensiblement verticale par rapport au sol (Fig. 5) et arrêtée lorsque la tête 27 n'est plus en position sensiblement verticale (Fig. 6), c'est-à-dire se trouve en position sensiblement horizontale par rapport au sol.

Plus précisément, le museau 3 du ruminant 2 s'étendant selon une direction B, la mesure est déclenchée lorsque la direction B du museau 3 fait un angle α par rapport à la direction verticale A au sol S, de préférence compris entre 0 et 45° (Fig. 5).

La mesure n'est pas déclenchée ou arrêtée lorsque l'angle α est supérieur à 45° (Fig. 6).

Ainsi, les quantités de méthane sont seulement mesurées lorsque le ruminant 2 penche ou incline la tête 27 vers le bas pour brouter de l'herbe. En effet, le méthane est émis seulement lorsque l'animal broute. Ceci permet d'augmenter l'autonomie de la batterie de l'appareil de mesure 5 et d'éviter d'augmenter la durée des mesures inutilement.

En variante, lorsque le ruminant 2 à la tête 27 baissée, la mesure est discontinue pour économiser la batterie.

Le processus d'acquisition peut comprendre des périodes de mesure suivies par des périodes de repos et des temps de chauffe de l'appareil de mesure 5.

En variante, la fréquence d'échantillonnage peut être programmée.

Lors de l'étape de mesure, l'appareil de mesure 5 enregistre la durée et la date des mesures.

Ces données sont stockées dans une mémoire prévue dans l'appareil de mesure 5 ou le boîtier électronique 28. Elles sont transmises à la base de données lorsque la mémoire est pleine ou à intervalles programmés.

L'appareil de mesure 5 peut également mesurer la température et l'hygrométrie.

## Revendications

1. Dispositif (1) pour mesurer les quantités de gaz rejetées lors des éructations d'un animal (2), le dispositif comprenant un appareil de mesure (5) des quantités de gaz rejetées par l'animal (2), des moyens de fixation (4) dudit dispositif (1) sur le museau (3) de l'animal (2), et une jupe (6) apte à être positionnée au-dessus du museau (3) de l'animal (2) et comprenant des moyens pour collecter le gaz ressortant par la bouche (26) de l'animal (2) et pour l'acheminer jusqu'à l'appareil de mesure (5), **caractérisé en ce que**:
- la jupe (6) présente une forme générale de U inversé et entoure seulement le dessus et les flancs du museau (3) de l'animal (2) tout en étant ouverte devant les nasaux et la bouche 26 et sous le museau.

2. Dispositif (1) selon l'une quelconques des revendication 1, **caractérisé en ce que** la jupe (6) comprend une partie supérieure arquée (7) prolongée par deux parties latérales (8) opposées, la partie supérieure arquée (7) s'étendant depuis le voisinage des yeux (9) de l'animal (2) jusqu'au voisinage de l'extrémité (10) du museau (3) du ruminant (2) et les deux parties latérales (8, 8') se prolongeant depuis la partie supérieure arquée (7) jusqu'au voisinage de la partie inférieure (11) du museau (3) de l'animal (2) lorsque le dispositif (1) est positionné sur l'animal (2).

3. Dispositif (1) selon la revendication 2, **caractérisé en ce que** la partie supérieure arquée (7) de la jupe (6) comprend un logement (12) destiné à recevoir l'appareil de mesure (5), la jupe (6) comprenant un orifice (13) débouchant dans le logement (12) et permettant de canaliser le gaz d'une zone inférieure (14) située en dessous de la jupe (6) vers le logement (12).

4. Dispositif (1) selon la revendication 3, **caractérisé en ce que** le logement (12) est délimité par une enveloppe de protection (15) comprenant une ouverture (16) pour l'introduction de l'appareil de mesure (5).

5. Dispositif (1) selon la revendication 4, **caractérisé en ce que** l'enveloppe de protection (15) comprend une sangle (17) fixée au voisinage de l'ouverture (16) de l'enveloppe de protection (15) pour maintenir l'appareil de mesure (5) dans le logement (12).

6. Dispositif (1) selon l'une quelconques des revendications 1 à 5, **caractérisé en ce qu'**il comprend une lèvre d'étanchéité (18) positionnée sur une surface interne (19) de la jupe (6), la lèvre d'étanchéité (18) longeant un rebord interne (20) de la jupe (6) positionné au voisinage des yeux (9) de l'animal (2), cette lèvre étant destinée à venir en appui sur le museau (3) de l'animal (2) pour empêcher le passage du gaz de la zone inférieure (14) située en dessous de la jupe (6) vers une zone supérieure positionnée au-dessus de la jupe (6).

7. Dispositif (1) selon l'une quelconques des revendications 1 à 6, **caractérisé en ce que** les moyens de fixation (4) comprennent un élément de fixation (25), tel qu'une ou plusieurs sangles (251, 252, 253), pour fixer le dispositif (1) sur la tête de l'animal (2), ainsi qu'un guide (35), pour cet élément de fixation (25), longeant le rebord interne (20) de la jupe (6) d'une extrémité d'une partie latérale (8) de la jupe (6) à l'autre (8').

8. Dispositif (1) selon la revendication 7, **caractérisé en ce que** l'élément de fixation (25) comprend une première sangle (251) qui est logée dans le guide (35) et dont les extrémités sont destinées à se rejoindre de manière à ce que ladite sangle (251) entoure le museau (3) de l'animal (2) et une seconde sangle dont deux portions (252, 254) s'étendent perpendiculairement à la première sangle (251) et sont prévues pour être reliées l'une à l'autre à l'arrière de la tête de l'animal (2).

9. Dispositif (1) selon l'une quelconques des revendications 1 à 8, **caractérisé en ce que** l'appareil de mesure (5) comprend un capteur résistif ou à infrarouge mesurant les quantités de gaz émises par l'animal (2).

10. Dispositif (1) selon l'une quelconques des revendications 1 à 9, **caractérisé en ce qu'**il comprend un boîtier électronique (28) relié électriquement à l'appareil de mesure (5) pour collecter les données de mesures enregistrées par l'appareil de mesure (5), le boîtier électronique (28) comprenant un moyen de communication sans fil pour transmettre lesdites données de mesures à une base de données distante, ledit boîtier électronique (28) étant destiné à être positionné sous le museau (3) de l'animal (2).

11. Dispositif (1) selon l'une quelconques des revendications 1 à 10, **caractérisé en ce qu'**il comprend un gyroscope et/ou un inclinomètre (29) pour déterminer l'inclinaison de la tête (27) de l'animal (2) afin de déclencher ou arrêter l'appareil de mesure (5).

12. Procédé pour mesurer les quantités de gaz rejetées lors des éructations d'un animal (2) au moyen d'un dispositif (1) tel que défini selon l'une quelconques des revendications 1 à 11, ledit dispositif (1) étant fixé sur le museau (3) de l'animal (2) par des moyens de fixation (4) et la jupe (6) étant positionnée au-dessus du museau (3) de l'animal (2) pour collecter le gaz rejeté par la bouche (26), l'appareil de mesure (5) étant disposé sur la jupe (6), ledit procédé étant **caractérisé en ce qu'**il comprend les étapes suivantes :
- collecte du gaz rejeté par l'animal (2) dans une zone inférieure (14) située sous la jupe (6),
- canalisation du gaz collecté de la zone inférieure (14) vers l'appareil de mesure (5), et
- mesure des quantités de gaz par l'appareil de mesure (5).

13. Procédé selon la revendication 12, **caractérisé en ce qu'**il comprend une étape de détection de l'inclinaison de la tête (27) de l'animal (2) pour déclencher ou arrêter la mesure.

14. Procédé selon la revendication 13, **caractérisé en ce que**, la détection de l'inclinaison de la tête (27) étant réalisée par un gyroscope et/ou inclinomètre positionné sur le dispositif (1), la mesure des quantités de gaz est déclenchée lorsque la tête (27) est en position sensiblement verticale et arrêtée lorsque la tête (27) est inclinée par rapport à la verticale.

## Patentansprüche

1. Vorrichtung (1) zum Messen der Gasmengen, die beim Aufstoßen eines Tieres (2) ausgestoßen werden, wobei die Vorrichtung ein Gerät (5) zum Messen der Gasmengen, die von dem Tier (2) ausgestoßen werden, Mittel (4) zum Befestigen der Vorrichtung (1) an der Schnauze (3) des Tieres (2) und eine Schürze (6), die über der Schnauze (3) des Tieres (2) positioniert werden kann und Mittel zum Sammeln des aus dem Mund (26) des Tieres (2) austretenden Gases und zu seinem Befördern bis zu dem Messgerät (5) umfasst, umfasst, **dadurch gekennzeichnet, dass**
- die Schürze (6) die allgemeine Form eines umgedrehten U aufweist und nur die Oberseite und die Flanken der Schnauze (3) des Tieres (2) umgibt, wobei sie vor den Nasenlöchern und dem Mund (26) und unter der Schnauze offen ist.

2. Vorrichtung (1) nach Anspruch 1, **dadurch gekennzeichnet, dass** die Schürze (6) einen bogenförmigen oberen Teil (7), der durch zwei gegenüberliegende laterale Teile (8) verlängert wird, umfasst, wobei sich der bogenförmige obere Teil (7) von der Nähe der Augen (9) des Tieres (2) bis zu der Nähe des Endes (10) der Schnauze (3) des Wiederkäuers (2) erstreckt und sich die beiden lateralen Teile (8, 8') von dem bogenförmigen oberen Teil (7) bis zu der Nähe des unteren Teils (11) der Schnauze (3) des Tieres (2) erstrecken, wenn die Vorrichtung (1) auf dem Tier (2) positioniert ist.

3. Vorrichtung (1) nach Anspruch 2, **dadurch gekennzeichnet, dass** der bogenförmige obere Teil (7) der Schürze (6) eine Aufnahme (12) umfasst, die das Messgerät (5) aufnehmen soll, wobei die Schürze (6) eine Öffnung (13) umfasst, die in der Aufnahme (12) mündet und gestattet, das Gas von einer unteren Zone (14), die sich unter der Schürze (6) befindet, zu der Aufnahme (12) zu leiten.

4. Vorrichtung (1) nach Anspruch 3, **dadurch gekennzeichnet, dass** die Aufnahme (12) durch einen Schutzmantel (15) begrenzt wird, der einen Durchlass (16) zum Einführen des Messgeräts (5) umfasst.

5. Vorrichtung (1) nach Anspruch 4, **dadurch gekennzeichnet, dass** der Schutzmantel (15) einen Gurt (17) umfasst, der in der Nähe des Durchlasses (16) des Schutzmantels (15) befestigt ist, um das Messgerät (5) in der Aufnahme (12) zu halten.

6. Vorrichtung (1) nach einem der Ansprüche 1 bis 5, **dadurch gekennzeichnet, dass** sie eine Dichtlippe (18) umfasst, die auf einer Innenfläche (19) der Schürze (6) positioniert ist, wobei sich die Dichtlippe (18) entlang einem inneren Rand (20) der Schürze (6), der in der Nähe der Augen (9) des Tieres (2) positioniert ist, erstreckt, wobei die Lippe an der Schnauze (3) des Tieres (2) in Anlage kommen soll, um das Passieren des Gases von der unteren Zone (14), die sich unter der Schürze (6) befindet, zu einer oberen Zone, die über der Schürze (6) positioniert ist, zu verhindern.

7. Vorrichtung (1) nach einem der Ansprüche 1 bis 6, **dadurch gekennzeichnet, dass** die Befestigungsmittel (4) ein Befestigungselement (25), wie zum Beispiel einen oder mehrere Gurte (251, 252, 253), zum Befestigen der Vorrichtung (1) an dem Kopf des Tieres (2) sowie eine Führung (35) für dieses Befestigungselement (25), die sich von einem Ende des lateralen Teils (8) der Schürze (6) zum anderen (8') entlang dem inneren Rand (20) der Schürze (6) erstreckt, umfassen.

8. Vorrichtung (1) nach Anspruch 7, **dadurch gekennzeichnet, dass** das Befestigungselement (25) einen ersten Gurt (251), der in der Führung (35) untergebracht ist und von dem die Enden zusammengeführt werden sollen, derart, dass der Gurt (251) die Schnauze (3) des Tieres (2) umgibt, und einen zweiten Gurt, von dem sich zwei Abschnitte (252, 254) senkrecht zu dem ersten Gurt (251) erstrecken und die dazu vorgesehen sind, hinter dem Kopf des Tieres (2) miteinander verbunden zu werden, umfasst.

9. Vorrichtung (1) nach einem der Ansprüche 1 bis 8, **dadurch gekennzeichnet, dass** das Messgerät (5) einen Widerstands- oder einen Infrarotsensor umfasst, der die von dem Tier (2) abgegebenen Gasmengen misst.

10. Vorrichtung (1) nach einem der Ansprüche 1 bis 9, **dadurch gekennzeichnet, dass** sie eine Elektronikeinheit (28) umfasst, die mit dem Messgerät (5) elektrisch verbunden ist, um die von dem Messgerät (5) aufgezeichneten Messdaten zu erfassen, wobei die Elektronikeinheit (28) ein drahtloses Kommunikationsmittel umfasst, um die Messdaten zu einer entfernten Datenbank zu übertragen, wobei die Elektronikeinheit (28) unter der Schnauze (3) des Tieres (2) positioniert werden soll.

11. Vorrichtung (1) nach einem der Ansprüche 1 bis 10, **dadurch gekennzeichnet, dass** sie ein Gyroskop und/oder einen Neigungsmesser (29) zum Bestimmen der Neigung des Kopfes (27) des Tieres (2) zwecks Aktivierens oder Deaktivierens des Messgeräts (5) umfasst.

12. Verfahren zum Messen der Gasmengen, die beim Aufstoßen eines Tieres (2) ausgestoßen werden, mittels einer Vorrichtung (1) wie gemäß einem der Ansprüche 1 bis 11 definiert, wobei die Vorrichtung (1) durch Befestigungsmittel (4) an der Schnauze (3) des Tieres (2) befestigt ist und die Schürze (6) über der Schnauze (3) positioniert ist, um das von dem Mund (26) des Tieres (2) ausgestoßene Gas zu sammeln, wobei das Messgerät (5) auf der Schürze (6) angeordnet ist, wobei das Verfahren **dadurch gekennzeichnet ist, dass** es die folgenden Schritte umfasst:
- Sammeln des von dem Tier (2) ausgestoßenen Gases in einer unteren Zone (14), die sich unter der Schürze (6) befindet,
- Leiten des gesammelten Gases von der unteren Zone (14) zu dem Messgerät (5) und
- Messen der Gasmengen mit dem Messgerät (5).

13. Verfahren nach Anspruch 12, **dadurch gekennzeichnet, dass** es einen Schritt des Detektierens der Neigung des Kopfes (27) des Tieres (2) zum Aktivieren oder Deaktivieren der Messung umfasst.

14. Verfahren nach Anspruch 13, **dadurch gekennzeichnet, dass** das Detektieren der Neigung des Kopfes (27) durch ein Gyroskop und/oder einen Neigungsmesser realisiert wird, das/der an der Vorrichtung (1) positioniert ist, wobei die Messung der Gasmengen aktiviert wird, wenn sich der Kopf (27) in einer im Wesentlichen vertikalen Position befindet, und deaktiviert wird, wenn der Kopf (27) bezüglich der Vertikalen geneigt ist.

## Claims

1. Device (1) for measuring the quantities of gas released when an animal (2) belches, the device comprising an instrument (5) for measuring the quantities of gas released by the animal (2), means (4) for fastening said device (1) to the muzzle (3) of the animal (2), and a skirt (6) that is able to be positioned above the muzzle (3) of the animal (2) and comprises means for collecting the gas escaping through the mouth (26) of the animal (2) and to carry it to the measuring instrument (5), **characterized in that**:
- the skirt (6) has the overall shape of an inverted U and surrounds only the top and sides of the muzzle (3) of the animal (2) while being open in front of the nostrils and the mouth (26) and under the muzzle.

2. Device (1) according to Claim 1, **characterized in that** the skirt (6) comprises an arched upper part (7) extended by two opposite lateral parts (8), the arched upper part (7) extending from the vicinity of the eyes (9) of the animal (2) to the vicinity of the end (10) of the muzzle (3) of the ruminant (2), and the two lateral parts (8, 8') extending from the arched upper part (7) to the vicinity of the lower part (11) of the muzzle (3) of the animal (2) when the device (1) is positioned on the animal (2).

3. Device (1) according to Claim 2, **characterized in that** the arched upper part (7) of the skirt (6) comprises a housing (12) intended to receive the measuring instrument (5), the skirt (6) comprising an orifice (13) that leads into the housing (12) and is intended to convey the gas from a lower zone (14) situated below the skirt (6) into the housing (12).

4. Device (1) according to Claim 3, **characterized in that** the housing (12) is delimited by a protective jacket (15) comprising an opening (16) for introducing the measuring instrument (5).

5. Device (1) according to Claim 4, **characterized in that** the protective jacket (15) comprises a strap (17), fastened in the vicinity of the opening (16) in the protective jacket (15), for keeping the measuring instrument (5) in the housing (12).

6. Device (1) according to any one of Claims 1 to 5, **characterized in that** it comprises a sealing lip (18) positioned on an internal surface (19) of the skirt (6), the sealing lip (18) following an internal rim (20) of the skirt (6) that is positioned in the vicinity of the eyes (9) of the animal (2), this lip being intended to bear on the muzzle (3) of the animal (2) in order to prevent the gas from passing from the lower zone (14) situated beneath the skirt (6) to an upper zone positioned above the skirt (6).

7. Device (1) according to any one of Claims 1 to 6, **characterized in that** the fastening means (4) comprise a fastening element (25), such as one or more straps (251, 252, 253), for fastening the device (1) to the head of the animal (2), and a guide (35), for this fastening element (25), following the internal rim (20) of the skirt (6) from one end of one lateral part (8) of the skirt (6) to the other (8').

8. Device (1) according to Claim 7, **characterized in that** the fastening element (25) comprises a first strap (251) which is housed in the guide (35) and the ends of which are intended to meet such that said strap (251) surrounds the muzzle (3) of the animal (2), and a second strap, two portions (252, 254) of which extend perpendicularly to the first strap (251) and are intended to be connected together behind the head of the animal (2) .

9. Device (1) according to any one of Claims 1 to 8, **characterized in that** the measuring instrument (5) comprises a resistive or infrared sensor measuring the quantities of gas emitted by the animal (2).

10. Device (1) according to any one of Claims 1 to 9, **characterized in that** it comprises an electronic box (28) electrically connected to the measuring instrument (5) in order to collect the measurement data recorded by the measuring instrument (5), the electronic box (28) comprising a wireless communication means for transmitting said measurement data to a remote database, said electronic box (28) being intended to be positioned under the muzzle (3) of the animal (2).

11. Device (1) according to any one of Claims 1 to 10, **characterized in that** it comprises a gyroscope and/or an inclinometer (29) for determining the inclination of the head (27) of the animal (2) in order to start or stop the measuring instrument (5).

12. Method for measuring the quantities of gas released when an animal (2) belches by means of a device (1) as defined in any one of Claims 1 to 11, said device (1) being fastened to the muzzle (3) of the animal (2) by fastening means (4), and the skirt (6) being positioned above the muzzle (3) of the animal (2) in order to collect the gas released through the mouth (26), the measuring instrument (5) being disposed on the skirt (6), said method being **characterized in that** it comprises the following steps of:
- collecting the gas released by the animal (2) in a lower zone (14) situated under the skirt (6),
- conveying the gas collected from the lower zone (14) to the measuring instrument (5), and
- measuring the quantities of gas with the measuring instrument (5).

13. Method according to Claim 12, **characterized in that** it comprises a step of detecting the inclination of the head (27) of the animal (2) in order to start or stop the measurement.

14. Method according to Claim 13, **characterized in that**, with the inclination of the head (27) being detected by a gyroscope and/or inclinometer positioned on the device (1), the measurement of the quantities of gas is started when the head (27) is in a substantially vertical position and stopped when the head (27) is inclined with respect to the vertical.
